Europäisches Patentamt

European Patent Office

Office européen des brevets

(19)

(11) EP 0 614 879 B1

(12) EUROPÄISCHE PATENTSCHRIFT

(45) Veröffentlichungstag und Bekanntmachung des Hinweises auf die Patenterteilung:
04.06.1997 Patentblatt 1997/23

(51) Int Cl.⁶: **C07C 201/04**, C07C 203/00

(21) Anmeldenummer: 94102781.5

(22) Anmeldetag: 24.02.1994

(54) **Verfahren zur Herstellung von C1-C4-Alkyl-nitriten**

Process for the preparation of C1-C4 alkyl nitrites

Procédé pour la préparation de nitrites d'alkyle en C1-C4

(84) Benannte Vertragsstaaten:
**BE CH DE ES FR GB IT LI NL**

(30) Priorität: 08.03.1993 DE 4307192

(43) Veröffentlichungstag der Anmeldung:
**14.09.1994 Patentblatt 1994/37**

(73) Patentinhaber: **BAYER AG**
**51368 Leverkusen (DE)**

(72) Erfinder:
• **Landscheidt, Heinz, Dr.**
**D-47057 Duisburg (DE)**
• **Wagner, Paul, Dr.**
**D-40597 Düsseldorf (DE)**
• **Kricsfalussy, Zoltan, Dr.**
**D-51375 Leverkusen (DE)**
• **Klausener, Alexander, Dr.**
**D-50670 Köln (DE)**

(56) Entgegenhaltungen:
EP-A- 0 057 143        EP-A- 0 076 217
EP-A- 0 310 191        US-A- 4 879 401

## Beschreibung

Die vorliegende Erfindung betrifft ein technisches Verfahren zur Herstellung von Alkylnitriten aus Distickstofftrioxid und niederen Alkanolen, wobei das Distickstofftrioxid als solches, als Gemisch mit Stickstoffmonoxid oder als Gemisch seiner Vorläufer Stickstoffmonoxid/Stickstoffdioxid bzw. Stickstoffmonoxid/Distickstofftetroxid eingesetzt wird. Es wird ohne Beteiligung von Sauerstoff gearbeitet. Das niedere Alkanol wird zu einem Teil im unteren Teil des Reaktors gemeinsam mit den Stickstoffoxiden eingesetzt und zum restlichen Teil im oberen Teil des Reaktors aufgegeben.

Alkylnitrite (Alkylester der salpetrigen Säure) finden vielfältige Verwendung, beispielsweise als Zusatzstoffe für Motoröle, als Stabilisatoren für ungesättigte organische Verbindungen, als Spasmolytika, als Reagenzien für Oximierungen, Nitrosierungen und Diazotierungen sowie als Zwischenprodukte für chemische Synthesen.

Es ist seit langem bekannt, daß man Alkylnitrite, darunter insbesondere Methylnitrit, durch Umsetzung von Distickstofftrioxid mit den korrespondierenden Alkanolen, darunter insbesondere Methanol, herstellen kann. Da Distickstofftrioxid über einen weiten Temperaturbereich hinweg mit Stickstoffmonoxid und Stickstoffdioxid im Gleichgewicht steht (vgl. nachfolgende Reaktionsgleichung (1)), läßt sich mit gleichem Erfolg ein äquimolares Gemisch beider Gase zum Einsatz bringen. Das im genannten Gleichgewicht bei prozeßtypischen Temperaturen stets vorhandene Stickstoffdioxid steht seinerseits im Gleichgewicht mit Distickstofftetroxid (vgl. nachfolgende Reaktionsgleichungen (1) und (5)). Somit können beispielsweise bei der Herstellung von Methylnitrit aus Distickstofftrioxid und Methanol bzw. aus Stickstoffmonoxid, Stickstoffdioxid und Methanol grundsätzlich die nachfolgend bezeichneten Reaktionen zwischen den beteiligten Komponenten ablaufen:

$$(1) \qquad NO_2 + NO \leftrightarrow N_2O_3$$

$$(2) \qquad R\text{-}OH + N_2O_3 \rightarrow R\text{-}ONO + HNO_2$$

$$(3) \qquad R\text{-}OH + HNO_2 \rightarrow R\text{-}ONO + H_2O$$

$$(4) \qquad N_2O_3 + H_2O \rightarrow 2\ HNO_2$$

$$(5) \qquad 2\ NO_2 \leftrightarrow N_2O_4$$

$$(6) \qquad R\text{-}OH + N_2O_4 \rightarrow R\text{-}ONO + HNO_3$$

$$(7) \qquad H_2O + N_2O_4 \rightarrow HNO_2 + HNO_3 \qquad (R\text{=}CH_3)$$

Bei der Herstellung der angestrebten Alkylnitrite geht man bevorzugt so vor, daß möglichst nur die in den Reaktionsgleichungen (1) bis (3) wiedergegebenen Umsetzungen ablaufen und als einziger Abfallstoff Wasser erhalten wird. Die in Reaktionsgleichung (4) wiedergegebene Reaktion ist im allgemeinen nicht vermeidbar, da das zudosierte bzw. nach Reaktionsgleichung (1) gebildete Distickstofftrioxid nicht nur mit dem Alkohol im Sinne der Reaktionsgleichung (2), sondern auch mit dem nach Reaktionsgleichung (3) entstandenen Wasser abreagieren kann. In Gegenwart ausreichender, insbesondere überschüssiger Mengen des Alkohols wird jedoch die dabei entstehende salpetrige Säure im Sinne der Reaktionsgleichung (3) unter Bildung des gewünschten Alkylnitrits und Wassers abgefangen, geht also nicht etwa als Abfallstoff verloren.

Die in den Reaktionsgleichungen (5) bis (7) wiedergegebenen Umsetzungen sind unerwünscht, da sie zur irreversiblen Bildung von Salpetersäure führen. Durch Bildung dieses Nebenproduktes wird die Ausbeute an gewünschtem Alkylnitrit, bezogen auf eingesetztes Stickoxid, verringert. Die entstandene Salpetersäure muß abgetrennt werden, und insbesondere bei Durchführung des Gesamtprozesses in technischem Maßstab fallen dabei Abwässer an, die unter Aufwendung nicht unerheblicher Kosten nachbehandelt werden müssen. Um die nach Reaktionsgleichung (5) ablaufende Dimerisierung des entweder im Gemisch mit Stickstoffmonoxid zudosierten oder über die Gleichgewichtsreaktion (1) gebildeten Stickstoffdioxids, die die Bildung der Salpetersäure einleitet, weitestgehend auszuschließen, stellt man das stöchiometrische Verhältnis von Stickstoffmonoxid zu Stickstoffdioxid bevorzugt so ein, daß es einen Wert > 1 annimmt. Auf diese Weise gelingt es, die Bildung von Distickstofftetroxid zugunsten der von Distickstofftrioxid zurückzudrängen.

Wie aus den Reaktionsgleichungen (1) bis (2) zu entnehmen ist, kann bei der Einspeisung von Stickoxiden zur Herstellung von Alkylnitriten vorteilhaft beispielsweise folgendermaßen vorgegangen werden:

- Man speist Stickstoffmonoxid und Stickstoffdioxid getrennt oder als Gemisch ein, und zwar bevorzugt in einem molaren Verhältnis $NO:NO_2$ von > 1, was entsprechend den obigen Ausführungen die Bevorzugung der Bildung von Distickstofftrioxid gegenüber der von Distickstofftetroxid und damit verbunden eine Zurückdrängung der Entstehung von Salpetersäure zur Folge hat.

- Man speist Distickstofftrioxid und Stickstoffmonoxid getrennt oder als Gemisch ein, wobei, wenn man Distickstofftrioxid als formal aus je einem Mol Stickstoffmonoxid und Stickstoffdioxid zusammengesetzt betrachtet, ein Verhältnis $NO:NO_2$ von > 1 vorliegt, was gemäß den obigen Ausführungen gleichfalls die Bildungswahrscheinlichkeit von Distickstofftetroxid verringert und damit verbunden eine Zurückdrängung der Entstehung von Salpetersäure zur Folge hat.

Wesentlich für die erfolgreiche Betreibung eines kontinuierlichen Prozesses zur Erzeugung von Methylnitrit ist die Berücksichtigung folgender Forderungen:

- Das zur Bildung des Alkylnitrits zugespeiste Distickstofftrioxid, Distickstofftrioxid/Stickstoffmonoxid-Gemisch bzw. Stickstoffmonoxid/Stickstoffdioxid-Gemisch oder Stickstoffmonoxid/Distickstofftetroxid-Gemisch soll möglichst vollständig zu Alkylnitrit umgesetzt werden.

- Die Reaktionsführung sowie die Reaktionstechnik sind so zu optimieren, daß die unerwünschte Bildung des Nebenproduktes Salpetersäure (vgl. Reaktionsgleichungen (6) und (7)) weitestgehend ausgeschlossen ist. Dies dient gleichzeitig einer maximal möglichen Ausbeute an gewünschtem Alkylnitrit.

- Die vollständige Umsetzung der zugespeisten Stickoxide zu dem gewünschten Alkylnitrit soll <u>innerhalb</u> des Alkylnitrit-Generators erfolgen. Damit wird vermieden, daß im Falle einer unvollständigen Abreaktion der beteiligten Komponenten innerhalb des vorgesehenen Reaktionsraumes eine vollständige Umsetzung erst <u>hinter</u> dem Alkylnitrit-Generators eintritt und daß das dabei entstehende Reaktionswasser (vgl. Reaktionsgleichung (3)) in eine etwaige nachgeschaltete Reaktion eingeschleppt wird, was dort gegebenenfalls zu unerwünschten Nebenreaktionen führen kann.

- Das im Verlaufe der Bildung des gewünschten Alkylnitrits entstehende Reaktionswasser sowie die gegebenenfalls infolge unerwünschter Nebenreaktionen gebildete Salpetersäure (vgl. Reaktionsgleichungen (6) und (7)) sollen möglichst vollständig aus dem gasförmigen Produktstrom, der den Alkylnitrit-Generator verläßt, abgetrennt werden.

- Die im Verlaufe der innerhalb des Alkylnitrit-Generators ablaufenden Reaktionen freiwerdende Reaktionswärme ist abzuführen.

- Im Hinblick auf die sicherheitstechnischen Anforderungen an die technische Durchführung von Prozessen, in die Alkylnitrit-Generatoren des hier genannten Prinzips integriert sind, müssen lokale Überhitzungen sowie das Entstehen zündfähiger Gemische vermieden werden.

Alle genannten Anforderungen werden durch das erfindungsgemäße Verfahren auf eine einfache, den Stand der Technik übertreffende Weise erfüllt.

In der Patentanmeldung EP 310 191, die ganz allgemein ein Verfahren für die Herstellung von Alkylnitriten, insbesondere von Methyl- und Ethylnitrit, beschreibt, wird unter Reaktionsbeteiligung von Sauerstoff vorgeschlagen, das gesamte Reaktionsgefäß, in dem die Reaktion zwischen Sauerstoff, Stickstoffmonoxid und dem jeweiligen Alkohol stattfindet, als Wäscher mit zwei räumlich getrennten Zonen, einer Reaktions- und einer Rektifikationszone, auszuführen. Das Waschmedium, in bevorzugter Weise identisch mit dem zur Reaktion gebrachten Alkohol und als solcher im stöchiometrischen Überschuß eingesetzt, wird nach dem Gegenstromprinzip am Kopf der Rektifikationszone eingespeist und dem aufsteigendem Produktgasstrom, bestehend unter anderem aus dem Alkylnitrit und dem bei dessen Bildung entstandenen Wasser, entgegengeführt. Nachteilig ist hierbei die getrennte Auslegung von Reaktions- und Rektifikationszone, die einen hohen apparativen Aufwand erfordert.

Der genannten Patentanmeldung zufolge wird die Rektifikationszone in Form einer Bodenkolonne ausgelegt. Unvorteilhaft bei einer derartigen Vorgehensweise ist, daß oberhalb eines Bodens jeweils eine völlig vermischte Gaszone vorliegt, wobei Rückvermischungseffekte auftreten. Um trotzdem den gewünschten Trenneffekt, d.h. die Entfernung des im Verlaufe der Bildung des Alkylnitrits entstandenen Wassers sowie der wasserlöslichen Nebenprodukte, wie beispielsweise Salpetersäure, zu realisieren, muß der gesamte Apparat deutlich größer dimensioniert werden, als dies bei Ausschluß von Rückvermischungseffekten nötig wäre. Es sei angemerkt, daß beispielsweise etwaige im den Alkylnitritgenerator verlassenden Produktgasstrom enthaltene Salpetersäure oder auch Wasser den nachgeschalteten Prozeß, beispielsweise die Herstellung von Oxalsäuredimethylester oder von Dimethylcarbonat, auch in kleinsten Mengen recht empfindlich stören können.

In der gleichen Patentanmeldung wird beschrieben, daß sich die bei der Entstehung des Alkylnitrits freiwerdende Reaktionswärme dadurch entfernen läßt, daß man aus der Reaktionszone einen flüssigen Seitenstrom abzieht, der nach externer Abkühlung in einen höher gelegenen Teil der Reaktionszone zurückgespeist wird. Dies hat jedoch neben einem erhöhten apparativen Aufwand den weiteren Nachteil, daß dadurch die Konzentration bzw. die Verweilzeit des gebildeten Reaktionswassers innerhalb der Reaktionszone erhöht bzw. verlängert werden. Dabei kann einerseits eine vermehrte Bildung von Nebenprodukten eintreten, andererseits werden der Einsatz gesteigerter Mengen an Waschflüssigkeit, speziell im Falle der Methylnitrit-Herstellung an Methanol, und gegebenenfalls eine weitere Vergrößerung der Rektifikationszone erforderlich, um das am Kopf des gesamten Reaktors austretende Produktgasgemisch weitestgehend wasserfrei zu erhalten.

Schließlich basieren alle Angaben, die der genannten Patentanmeldung EP 310 191 zu entnehmen sind, lediglich auf Computersimulationsrechnungen. Das einzige, gleichfalls auf einer derartigen Computersimulation basierende Beispiel ist für den Fachmann nicht nachvollziehbar, da erstens die ein- und ausgehenden Stoffströme, bezogen auf den Alkylnitritreaktor, nicht spezifiziert werden und zweitens keine Angaben über die Reaktionsvolumina, die allein über die durch die gegebene Reaktionskinetik erforderliche Verweilzeit entscheiden, gemacht werden. Die tatsächliche Effizienz der beschriebenen apparativen Anordnung ist demnach unzureichend belegt und kann daher überhaupt nicht beurteilt werden. Des weiteren ist das genannte Beispiel auch unter technischen Gesichtspunkten irrelevant, da es offenbar keine Inertisierung des grundsätzlich zündfähigen Alkylnitritsstromes beinhaltet und daher die bei sicherheitstechnisch hinreichender Fahrweise auftretende durch den Inertgasanteil bedingte Schleppwirkung keine Berücksichtigung findet. Da sich beispielsweise die untere Explosionsgrenze des Systems Alkylnitrit/Stickstoffmonoxid/Kohlenmonoxid/Alkohol/Inertgas bei steigendem Druck zu kleineren Alkylnitritkonzentrationen hin verschiebt, kommt dieser Frage insbesondere bei Kreisprozessen, wie beispielsweise der technischen Herstellung von Oxalsäuredimethylester oder Dimethylcarbonat, bei denen derartige Gasgemische in Alkylnitrit-Reaktoren eingespeist werden, eine ganz entscheidende Bedeutung zu.

Es bestand daher nach wie vor die Aufgabe, ein Verfahren zur kontinuierlichen Herstellung von $C_1$-$C_4$-Alkyl-nitriten aus den zugrunde liegenden $C_1$-$C_4$-Alkanolen und Stickstoffoxiden zu finden, das die obengenannte Forderungen berücksichtigt und sich für die Integration in kontinuierliche Prozesse eignet, bei denen $C_1$-$C_4$-Alkyl-nitrite verbraucht werden.

Die Erfindung betrifft ein Verfahren zur Herstellung von $C_1$-$C_4$-Alkyl-nitriten durch Umsetzung von $C_1$-$C_4$-Alkanolen mit Stickstoffoxiden ohne Beteiligung von Sauerstoff in einem als Wäscherkolonne ausgebildeten Reaktor, das dadurch gekennzeichnet ist, daß als Stickstoffoxid Distickstofftrioxid im unteren Teil des Reaktors eingesetzt wird und zwar

a) als solches,

b) als Gemisch mit Stickstoffmonoxid oder

c) als Gemisch seiner Vorläufer Stickstoffmonoxid/Stickstoffdioxid bzw. Stickstoffmonoxid/Distickstofftetroxid,

daß das Distickstofftrioxid mit einem oder mehreren Inertgasen, deren Anteil 0 bis 90 Vol-% aller eingesetzten Gase beträgt, verdünnt sein kann, daß das $C_1$-$C_4$-Alkanol zu 5 bis 60 % seiner gesamten Einsatzmenge ebenfalls im unteren Teil des Reaktors in dampfförmiger oder verdüster Form eingesetzt wird, während das restliche $C_1$-$C_4$-Alkanol auf den Reaktorkopf gegeben wird, wobei die gesamte Einsatzmenge an $C_1$-$C_4$-Alkanol 0,8 bis 2 Mol je Grammatom N im Distickstofftrioxid beträgt und wobei bei 10 bis 150°C und 0,5 bis 6 bar gearbeitet wird und die Verweilzeit der Reaktionspartner im Reaktor auf 1 bis 500 sec. eingestellt wird.

$C_1$-$C_4$-Alkanole für das erfindungsgemäße Verfahren sind beispielsweise Methanol, Ethanol, Propanol, Isopropanol, Butanol, sek.-Butanol, i-Butanol, bevorzugt Methanol und Ethanol, besonders bevorzugt Methanol. Demzufolge werden die davon abgeleiteten $C_1$-$C_4$-Alkylnitrite, bevorzugt Methylnitrit und Ethylnitrit, besonders bevorzugt Methylnitrit hergestellt.

Das erfindungsgemäße Verfahren arbeitet ohne Reaktionsbeteiligung von Sauerstoff.

Es wird erfindungsgemäß angestrebt, als Reaktionspartner des $C_1$-$C_4$-Alkanols Distickstofftrioxid einzusetzen, wie es den obigen Gleichungen (2), (3) und (4) entspricht.

Somit kann Stickstofftrioxid als solches eingesetzt werden oder als Gemisch mit Stickstoffmonoxid zur Zurückdrängung unerwünschter Reaktionen gemäß obiger Erläuterung oder schließlich als Gemisch der Vorläufer Stickstoffmonoxid/Stickstoffdioxid bzw. Stickstoffmonoxid/Stickstofftetroxid, die jeweils zu Distickstofftrioxid reagieren können (beispielsweise nach Gleichung (1)). In bevorzugter Weise wird ein Gemisch aus dem technisch gut zugänglichen und verfügbaren Vorläufern des Distickstofftrioxids, nämlich ein Stickstoffmonoxid/Stickstoffdioxid-Gemisch bzw. ein Stickstoffmonoxid/Distickstofftetroxid-Gemisch eingesetzt.

Während es grundsätzlich auch möglich ist, eine unterstöchiometrische Menge an Stickstoffmonoxid zu benutzen und die dabei auftretenden unerwünschten Nebenreaktionen gemäß obiger Darstellung in einem geringen Maße zu tolerieren, ist es bevorzugt, mindestens äquimolare Mengen an Stickstoffmonoxid oder einen überproportionalen molaren Anteil an Stickstoffmonoxid im Gemisch vorliegen zu haben. Bei der bevorzugten Variante des Einsatzes von $NO/NO_2$-Gemischen oder $NO/N_2O_4$-Gemischen liegt daher das NO in einer Menge von 45 bis 95 Mol-%, bevorzugt 50 bis 90 Mol-%, besonders bevorzugt 70 bis 85 Mol-% aller im Gemisch vorhandenen Stickstoffoxide vor. Bei dieser molaren Berechnung wird $N_2O_4$ als 2 Mole $NO_2$ gerechnet.

Die erfindungsgemäß einzusetzenden Stickstoffoxide oder Stickstoffoxid-Gemische können grundsätzlich ohne Vermischung mit inerten Gasen eingesetzt werden. In bevorzugter Weise wird jedoch ein Inertgas oder ein Gemisch mehrerer von ihnen zugesetzt. Der Anteil des oder der Inertgas(e) beträgt daher 0 bis 90 Vol-% aller eingesetzten Gase, bevorzugt 10 bis 80 Vol-%, besonders bevorzugt 20 bis 70 Vol-%. Als Inertgase kommen hierbei alle in Frage, die mit den Ausgangsstoffen oder den Reaktionsprodukten keine chemischen Reaktionen eingehen. In bevorzugter Weise handelt es sich dabei um eines oder mehrere Gase aus der Gruppe von $CO_2$, $N_2$, Ar, He, bevorzugt aus der Gruppe von $CO_2$ und $N_2$.

Es ist ein wesentliches Kennzeichen des erfindungsgemäßen Verfahrens, daß das umzusetzende $C_1$-$C_4$-Alkanol an zwei Stellen im Reaktor eingesetzt wird. 5 bis 60 % seiner gesamten Einsatzmenge, bevorzugt 10 bis 50 %, besonders bevorzugt 10 bis 30 % des $C_1$-$C_4$-Alkanols werden im unteren Teil des Reaktors in verdampfter oder verdüster Form eingesetzt, während das restliche $C_1$-$C_4$-Alkanol auf den Reaktorkopf gegeben wird. In bevorzugter Weise wird der im unteren Teil des Reaktors eingesetzte Anteil an $C_1$-$C_4$-Alkanol in verdüster Form eingesetzt; der mittlere Tropfendurchmesser beträgt hierbei 5 bis 1.000 µm, bevorzugt 5 bis 500 µm.

Es ist weiterhin eine bevorzugte Variante, beide Ausgangsstoffströme, die erfindungsgemäß im unteren Teil des Reaktors eingesetzt werden, nämlich das $C_1$-$C_4$-Alkanol und den Stickstoffoxidstrom, gegebenenfalls im Gemisch mit Inertgasen, als einen gemeinsamen Stoffstrom einzusetzen. In einem solchen Fall kann in Kombination mit der bevorzugten Verdüsung des $C_1$-$C_4$-Alkanol-Anteils der Strom der Einsatzgase (Stickstoffoxid, gegebenenfalls mit Inertgas) zur Verdüsung des Alkanol-Anteils herangezogen werden.

Die gesamte Einsatzmenge an $C_1$-$C_4$-Alkanol, also sowohl der im unteren Teil des Reaktors als auch im oberen Teil des Reaktors eingesetzte Teil, betragen gemeinsam 0,8 bis 2 Mol je Grammatom Stickstoff im Distickstofftrioxid (bzw. seinen Vorläufer-Gemischen), bevorzugt 1 bis 1,5 Mol, besonders bevorzugt 1 bis 1,2 Mol. Der gesamte Bereich umfaßt also einen geringfügig unterstöchiometrischen Anteil, innerhalb dessen die unerwünschten Nebenreaktionen bis zu einem gewissen Ausmaße toleriert werden, bis zu einem überstöchiometrischen Anteil, bevorzugt jedoch einen nahezu äquimolaren Anteil oder wenig darüber.

Das erfindungsgemäße Verfahren wird bei einer Temperatur von 10 bis 150°C, bevorzugt bei 30 bis 100°C, und bei einem Druck von 0,5 bis 6 bar, bevorzugt 0,8 bis 5 bar, besonders bevorzugt 1 bis 4 bar, durchgeführt, wobei eine Verweilzeit der Reaktionspartner im Reaktor auf 1 bis 500 sec., bevorzugt 5 bis 300 sec., besonders bevorzugt 10 bis 50 sec. eingestellt wird. Temperatur, Druck und Verweilzeit können grundsätzlich unabhängig voneinander eingestellt werden.

Der Reaktor für das erfindungsgemäße Verfahren ist ein als Wäscherkolonne ausgebildeter Reaktor. Solche Kolonnen sind mit Einbauten versehen, die grundsätzlich bekannt sind und für eine innige Flüssig-Gas-Vermischung sorgen. Solche Einbauten sind im wesentlichen die gleichen, die auch bei der Durchführung thermischer Trennoperationen gebräuchlich sind, beispielsweise Böden mit Glocken, Sieblochböden, Ventilböden, Schlitzböden und weitere dem Fachmann bekannte; ferner können Füllkörper aller Art, mit geordneten Packungen, bevorzugt mit strukturierten Packungen, eingesetzt werden; ferner kommen weitere Einbauten wie Umlenkbleche und Schikanebleche in Frage.

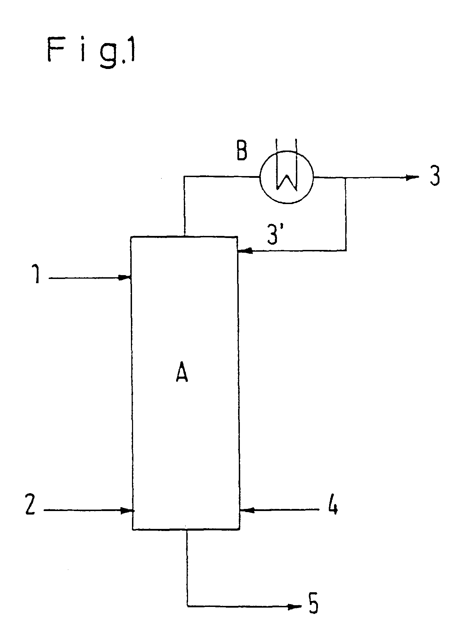

Das erfindungsgemäße Verfahren kann beispielhaft mit Hilfe der beigefügten Figur 1 erläutert werden: Figur 1 zeigt einen als Wäscherkolonne ausgebildeten Reaktor A mit einem am Kopf von A angebrachten Kühler B. Das umzusetzende Alkanol wird an den beiden Stellen (1) am Kopf des Reaktors und (2) im unteren Teil des Reaktors eingesetzt. Der Alkanolstrom bei (2) wird in verdampfter oder verdüster Form eingesetzt. Bei (4) wird der gasförmige Strom der Stickstoffoxide in der oben beschriebenen Weise, gegebenenfalls vermischt mit Inertgas, eingesetzt. Der Produktgasstrom wird am Kopf des Reaktors entnommen und durch den Kühler B geleitet. In diesem Kühler werden kondensierbare Anteile des Produktstromes, beispielsweise mitgerissenes Alkanol kondensiert und auf den Kopf der Kolonne, bevorzugt oberhalb der Alkanoleinspeisung (1) zurückgeführt. Der Rest wird über (3) entnommen. Am Sumpf der Kolonne wird Reaktionswasser (5) entnommen. Dieses Reaktionswasser enthält mitentstandene Salpetersäure sowie überschüssig eingesetztes Alkanol.

Zum Verdüsen des Alkanols bei (2) kann Inertgas der oben beschriebenen Art verwendet werden. In der bevorzugten, oben beschriebenen Variante der Zusammenfassung der Einsatzströme (2) und (4) kann der Gasstrom der Stickstoffoxide und des Inertgases zum Verdüsen des Alkanols eingesetzt werden.

Das erfindungsgemäße Verfahren besitzt verschiedene Vorzüge:

- Die Reaktionsführung sowie die Reaktionstechnik können so optimiert werden, daß die unerwünschte Bildung des Nebenproduktes Salpetersäure weitestgehend ausgeschlossen ist. Dadurch wird gleichzeitig eine maximale Ausbeute an gewünschtem Alkylnitrit erreicht. Überraschenderweise stellt es sich als besonders vorteilhaft für die angestrebte Minimierung der unerwünschten Bildung von Salpetersäure heraus, wenn das eingespeiste Reaktandgas, namentlich das Stickstoffoxidgemisch bzw. die einzelnen Stickoxidspezies, tunlichst unmittelbar nach ihrer Zusammenführung und bei Eintritt in den Reaktor bereits mit einer geeigneten Menge Alkanol unter möglichst schneller und vollständiger Vermischung aller Komponenten in Kontakt gebracht wird. Genau dies wird durch die bevorzugte Verwendung einer Düse für diese Operation, im erfindungsgemäßen Zusammenhang mit der Einspeisung eines Teils des Methanols in den unteren Bereich des Reaktors, bewirkt.

- Die vollständige Umsetzung der eingespeisten Stickoxide zu dem gewünschten Alkylnitrit erfolgt innerhalb des Alkylnitrit-Generators. Damit wird vermieden, daß im Falle einer unvollständigen Abreaktion der beteiligten Komponenten innerhalb des vorgesehenen Reaktionsraumes eine vollständige Umsetzung erst hinter dem Alkylnitrit-Generators eintritt und daß das dabei entstehende Reaktionswasser in eine etwaige nachgeschaltete Reaktion, bei der das hergestellte Alkylnitrit weiter umgesetzt wird, eingeschleppt wird, wodurch gegebenenfalls unerwünschte Nebenreaktionen verursacht werden können. Sollte es dennoch gewünscht sein, einen unvollständigen Umsatz an Stickoxiden zu erzielen und bespielsweise ein Produktgasgemisch zu erhalten, in dem noch unumgesetztes Stickstoffmonoxid vorhanden ist, so läßt sich dies nach dem erfindungsgemäßen Verfahren gleichfalls realisieren, beispielsweise durch eine Überdosierung des zum Einsatz gebrachten Stickstoffmonoxids.

- Das im Verlaufe der Bildung des gewünschten Alkylnitrits entstehende Reaktionswasser sowie die gegebenenfalls infolge unerwünschter Nebenreaktionen gebildete Salpetersäure werden praktisch vollständig aus dem gasförmigen Produktstrom, der den Alkylnitrit-Generator verläßt, abgetrennt.

- Die im Verlaufe der innerhalb des Alkylnitrit-Generators ablaufenden Reaktionen freiwerdende Reaktionswärme wird vollständig abgeführt. In überraschender Weise gelingt dies besonders glatt, wenn bei der erfindungsgemäßen und gemeinsam mit der Zuleitung des gasförmigen Reaktanden, namentlich der zum Einsatz kommenden Stickoxide, erfolgenden Einspeisung eines Teils des Alkanols in den unteren Bereich des Reaktionsgefäßes dieses Methanol in flüssiger Form eingebracht wird (verdüst statt verdampft).

- Im Hinblick auf die sicherheitstechnischen Anforderungen an die technische Durchführung von Prozessen, in die Alkylnitrit-Generatoren des hier genannten Prinzips integriert sind, werden lokale Überhitzungen sowie das Entstehen zündfähiger Gemische vermieden.

Beispiele

Beispiel 1

Herstellung von Methylnitrit aus Stickstoffmonoxid, Stickstoffdioxid und Methanol

Es wird ein Reaktorsystem verwendet, wie es in Figur 1 schematisch dargestellt ist (Volumen des Reaktionsteils der Kolonne 7,2 l; 9 theoretische Trennstufen, Kopfdruck 3030 mbar, ausgerüstet mit einem am Kopf der Kolonne angebrachten Wärmeaustauscher). In der Tabelle sind die zugespeisten bzw. abgeleiteten Ströme mit den aus Figur 1 entnommenen Ziffern gekennzeichnet. Strom 1, 2 und 5 sind flüssig (fl.), und Strom 3 und 4 sind gasförmig (g.). Während Strom 1, 2 und 4 die eingesetzten Reaktanden spezifizieren und quantifizieren, sind die sich experimentell ergebenden Gehalte der aufgelisteten Komponenten im Produktgasstrom als Strom 3 und im Ablauf als Strom 5 wiedergegeben.

| Strom 1 (fl.) | Strom 2 (fl.) | Strom 3 (g.) | | Strom 4 (g.) | | Strom 5 (fl.) | |
|---|---|---|---|---|---|---|---|
| MeOH 2009 g/h | MeOH 502 g/h | $CO_2$ | 3481 g/h | NO | 1012 g/h | MeOH | 63 g/h |
| | | NO | 78 g/h | $CO_2$ | 3481 g/h | $HNO_3$ | 40 g/h |
| | | MeOH | 432 g/h | $NO_2$ | 1491,0 g/h | Wasser | 559 g/h |
| | | MeONO | 3840 g/h | | | | |
| | | Wasser | <2,0 g/h | | | | |
| | | $HNO_3$ | 0 g/h | | | | |

Das Produktgasgemisch (Strom 3) hat beim Austritt aus dem Wärmeaustauscher eine Temperatur von 35°C.

$NO_2$ > 50 mol-%

EP 0 614 879 B1

**Patentansprüche**

1. Verfahren zur Herstellung von $C_1$-$C_4$-Alkyl-nitriten durch Umsetzung von $C_1$-$C_4$-Alkanolen mit Stickstoffoxiden ohne Beteiligung von Sauerstoff in einem als Wäscherkolonne ausgebildeten Reaktor, dadurch gekennzeichnet, daß als Stickstoffoxid Distickstofftrioxid im unteren Teil des Reaktors eingesetzt wird und zwar

    a) als solches,

    b) als Gemisch mit Stickstoffmonoxid oder

    c) als Gemisch seiner Vorläufer Stickstoffmonoxid/ Stickstoffdioxid bzw. Stickstoffmonoxid/Distickstofftetroxid,

    daß das Distickstofftrioxid mit einem oder mehreren Inertgasen, deren Anteil 0 bis 90 Vol-% aller eingesetzten Gase beträgt, verdünnt sein kann, daß das $C_1$-$C_4$-Alkanol zu 5 bis 60 % seiner gesamten Einsatzmenge ebenfalls im unteren Teil des Reaktors in dampfförmiger oder verdüster Form eingesetzt wird, während das restliche $C_1$-$C_4$-Alkanol auf den Reaktorkopf gegeben wird, wobei die gesamte Einsatzmenge an $C_1$-$C_4$-Alkanol 0,8 bis 2 Mol je Grammatom N im Distickstofftrioxid beträgt und wobei bei 10 bis 150°C und 0,5 bis 6 bar gearbeitet wird und die Verweilzeit der Reaktionspartner im Reaktor auf 1 bis 500 sec. eingestellt wird.

2. Verfahren nach Anspruch 1, dadurch gekennzeichnet, daß das Distickstofftrioxid als Gemisch seiner Vorläufer Stickstoffmonoxid/Stickstoffdioxid bzw. Stickstoffmonoxid/Distickstofftetroxid eingesetzt wird, wobei in diesen Vorläufergemischen Stickstoffmonoxid 45 bis 95 mol-%, bevorzugt 50 bis 90 mol-%, besonders bevorzugt 70 bis 85 mol-% aller Stickstoffoxide darstellt, wobei Distickstofftetroxid als 2 Mole Stickstoffdioxid gerechnet wird.

3. Verfahren nach Anspruch 1, dadurch gekennzeichnet, daß als Inertgas eines oder mehrere aus der Gruppe $CO_2$, $N_2$, Ar und He, bevorzugt $N_2$ und $CO_2$, verwendet werden und der Inertgasanteil 10 bis 80 Vol-%, bevorzugt 20 bis 70 Vol-% aller eingesetzten Gase beträgt.

4. Verfahren nach Anspruch 1, dadurch gekennzeichnet, daß der im unteren Teil des Reaktors eingesetzte Teil des $C_1$-$C_4$-Alkanols 10 bis 50 %, bevorzugt 10 bis 30 % der gesamten Einsatzmenge beträgt.

5. Verfahren nach Anspruch 1, dadurch gekennzeichnet, daß die gesamte Einsatzmenge an $C_1$-$C_4$-Alkanol 1 bis 1,5 mol, bevorzugt 1 bis 1,2 mol je Grammatom N im Distickstofftrioxid beträgt.

6. Verfahren nach Anspruch 1, dadurch gekennzeichnet, daß der im unteren Teil des Reaktors eingesetzte Anteil an $C_1$-$C_4$-Alkanol in verdüster Form eingesetzt wird, wobei der mittlere Tropfendurchmesser 5 bis 1000 µm, bevorzugt 5 bis 500 µm beträgt.

7. Verfahren nach Anspruch 1, dadurch gekennzeichnet, daß im unteren Teil des Reaktors als gemeinsamer Stoffstrom $C_1$-$C_4$-Alkanol-Anteil, Stickstoffoxid(e) und gegebenenfalls Inertgas(e) eingesetzt werden.

8. Verfahren nach Anspruch 7, dadurch gekennzeichnet, daß das (die) Stickstoffoxid(e) und das (die) gegebenenfalls miteingesetzte(n) Inertgas(e) zur Verdüsung des Anteils an $C_1$-$C_4$-Alkanol benutzt wird (werden).

9. Verfahren nach Anspruch 1, dadurch gekennzeichnet, daß bei einem Druck von 0,8 bis 6 bar, bevorzugt 1 bis 4 bar gearbeitet wird und unabhängig vom Druck bei 30 bis 100°C gearbeitet wird und unabhängig von Druck und Temperatur eine Verweilzeit von 5 bis 300 sec, bevorzugt 10 bis 50 sec eingestellt wird.

10. Verfahren nach Anspruch 1, dadurch gekennzeichnet, daß zur Herstellung von $C_1$-$C_2$-Alkyl-nitrit ein $C_1$-$C_2$-Alkanol umgesetzt wird und bevorzugt zur Herstellung von Methylnitrit Methanol umgesetzt wird.

**Claims**

1. Process for the preparation of $C_1$-$C_4$-alkyl nitrites by reaction of $C_1$-$C_4$-alkanols with nitrogen oxides without the participation of oxygen in a reactor designed as a scrubber column, characterized in that the nitrogen oxide used in the lower part of the reactor is dinitrogen trioxide, more precisely

a) as such,

b) as a mixture with nitrogen monoxide or

c) as a mixture of its precursors nitrogen monoxide/ nitrogen dioxide or nitrogen monoxide/dinitrogen tetroxide,

in that the dinitrogen trioxide can be diluted with one or more inert gases, the proportion of which is 0 to 90% by volume of all gases used, in that 5 to 60% of the total amount of the $C_1$-$C_4$-alkanol used is likewise charged in the lower part of the reactor in vaporous or atomized form, whereas the remaining $C_1$-$C_4$-alkanol is fed to the top of the reactor, the total amount of $C_1$-$C_4$-alkanol used being 0.8 to 2 mol per gram atom of N in the dinitrogen trioxide, 10 to 150°C and 0.5 to 6 bar being employed and the residence time of the reaction partners in the reactor being set at 1 to 500 sec.

2. Process according to Claim 1, characterized in that the dinitrogen trioxide is used as a mixture of its precursors nitrogen monoxide/nitrogen dioxide or nitrogen monoxide/dinitrogen tetroxide, nitrogen monoxide comprising 45 to 95 mol%, preferably 50 to 90 mol%, particularly preferably 70 to 85 mol%, of all nitrogen oxides in these precursor mixtures, dinitrogen tetroxide being calculated as 2 mol of nitrogen dioxide.

3. Process according to Claim 1, characterized in that the inert gas used is one or more selected from the group consisting of $CO_2$, $N_2$, Ar and He, preferably $N_2$ and $CO_2$, and the inert gas proportion is 10 to 80% by volume, preferably 20 to 70% by volume, of all gases used.

4. Process according to Claim 1, characterized in that the portion of the $C_1$-$C_4$-alkanol charged in the lower part of the reactor is 10 to 50%, preferably 10 to 30%, of the entire amount used.

5. Process according to Claim 1, characterized in that the entire amount of $C_1$-$C_4$-alkanol used is 1 to 1.5 mol, preferably 1 to 1.2 mol, per gram atom of N in the dinitrogen trioxide.

6. Process according to Claim 1, characterized in that the proportion of $C_1$-$C_4$-alkanol charged in the lower part of the reactor is charged in atomized form, the mean droplet diameter being 5 to 1000 µm, preferably 5 to 500 µm.

7. Process according to Claim 1, characterized in that a $C_1$-$C_4$-alkanol proportion, nitrogen oxide(s) and, if appropriate, inert gas(es) are charged in the lower part of the reactor as a joint material stream.

8. Process according to Claim 7, characterized in that the nitrogen oxide(s) and the inert gas(es) used conjointly if appropriate is (are) utilized to atomize the proportion of $C_1$-$C_4$-alkanol.

9. Process according to Claim 1, characterized in that a pressure of 0.8 to 6 bar, preferably 1 to 4 bar, is employed and, independently of the pressure, 30 to 100°C is employed and, independently of pressure and temperature, a residence time of 5 to 300 sec, preferably 10 to 50 sec, is set.

10. Process according to Claim 1, characterized in that a $C_1$-$C_2$-alkanol is reacted for the preparation of $C_1$-$C_2$-alkyl nitrite and, preferably, methanol is reacted for the preparation of methyl nitrite.

**Revendications**

1. Procédé pour la préparation de nitrites d'alkyle en $C_1$-$C_4$ par mise en réaction d'alcanols en $C_1$-$C_4$ avec des oxydes d'azote sans implication d'oxygène dans un réacteur réalisé sous forme d'une colonne de lavage, caractérisé en ce qu'on introduit, comme oxyde d'azote, de l'anhydride nitreux dans la partie inférieure du réacteur, à savoir

a) comme tel,

b) sous forme d'un mélange avec du monoxyde d'azote ou

c) sous forme d'un mélange de ses précurseurs monoxyde d'azote/dioxyde d'azote, respectivement monoxyde d'azote/peroxyde d'azote,

en ce que l'anhydride nitreux peut être dilué avec un ou plusieurs gaz inertes dont la fraction représente de 0 à 90% en volume de tous les gaz mis en oeuvre, en ce qu'on introduit l'alcanol en $C_1$-$C_4$ jusqu'à concurrence de 5 à 60% de sa quantité introduite totale, également dans la partie inférieure du réacteur, sous forme évaporée ou sous forme atomisée, tandis que le reste de l'alcanol en $C_1$-$C_4$ est ajouté à la tête du réacteur, dans lequel la quantité introduite totale d'alcanol en $C_1$-$C_4$ s'élève de 0,8 à 2 moles par atome-gramme de N dans l'anhydride nitreux et dans lequel on travaille à une température de 10 à 150°C et sous une pression de 0,5 à 6 bar, et on règle le temps de séjour des partenaires réactionnels dans le réacteur à un temps de 1 à 500 sec.

2. Procédé selon la revendication 1, caractérisé en ce qu'on met en oeuvre l'anhydride nitreux sous forme d'un mélange de ses précurseurs monoxyde d'azote/dioxyde d'azote, respectivement monoxyde d'azote/peroxyde d'azote, dans lequel, dans ces mélanges de précurseurs, le monoxyde d'azote représente de 45 à 95 moles %, de préférence de 50 à 90 moles %, de manière particulièrement préférée de 70 à 85 moles % de tous les oxydes d'azote, le peroxyde d'azote étant calculé comme 2 moles de dioxyde d'azote.

3. Procédé selon la revendication 1, caractérisé en ce qu'on utilise, comme gaz inerte, un ou plusieurs gaz choisis parmi le groupe comprenant $CO_2$, $N_2$, Ar et He, de préférence $N_2$ et $CO_2$, la fraction du gaz inerte représentant de 10 à 80% en volume, de préférence de 20 à 70% en volume de tous les gaz mis en oeuvre.

4. Procédé selon la revendication 1, caractérisé en ce que la partie de l'alcanol en $C_1$-$C_4$ introduite dans la partie inférieure du réacteur représente de 10 à 50%, de préférence de 10 à 30% de la quantité introduite totale.

5. Procédé selon la revendication 1, caractérisé en ce que la quantité introduite totale d'alcanol en $C_1$-$C_4$ s'élève de 1 à 1,5 mole, de préférence de 1 à 1,2 mole par atome-gramme de N dans l'anhydride nitreux.

6. Procédé selon la revendication 1, caractérisé en ce que la fraction d'alcanol en $C_1$-$C_4$ introduite dans la partie inférieure du réacteur est introduite sous forme atomisée, le diamètre moyen des gouttes s'élevant de 5 à 1000 μm, de préférence de 5 à 500 μm.

7. Procédé selon la revendication 1, caractérisé en ce qu'on introduit, dans la partie inférieure du réacteur, sous forme de courant de substances conjoint, une fraction de l'alcanol en $C_1$-$C_4$, du ou des oxydes d'azote et, le cas échéant, un ou des gaz inertes.

8. Procédé selon la revendication 7, caractérisé en ce qu'on utilise le ou les oxydes d'azote et le ou les gaz inertes éventuellement introduits de manière conjointe pour l'atomisation de la fraction d'alcanol en $C_1$-$C_4$.

9. Procédé selon la revendication 1, caractérisé en ce qu'on travaille sous une pression de 0,8 à 6 bar, de préférence de 1 à 4 bar, et on travaille, indépendamment de la pression, à une température de 30 à 100°C, et on règle, indépendamment de la pression et de la température, un temps de séjour de 5 à 300 sec, de préférence de 10 à 50 sec.

10. Procédé selon la revendication 1, caractérisé en ce que, pour la préparation de nitrites d'alkyle en $C_1$-$C_2$, on fait réagir un alcanol en $C_1$-$C_2$ et, de préférence, pour la préparation du nitrite de méthyle, on fait réagir du méthanol.

F i g.1